# EUROPEAN PATENT SPECIFICATION

(11) **EP 1 515 959 B1**
(45) Date of publication and mention of the grant of the patent: **12.08.2009**
(21) Application number: 03725195.6
(22) Date of filing: 04.06.2003
(51) Int. Cl.: C07D 265/30, A61K 31/5375, A61P 25/24

(54) **PHARMACEUTICAL SALTS OF REBOXETINE**
PHARMAZEUTISCHE SALZE VON REBOXETIN
SELS PHARMACEUTIQUES DE REBOXETINE

(30) Priority: 17.06.2002 EP 02077366
(43) Date of publication of application: 23.03.2005
(73) Proprietor: Pfizer Italia S.r.l., 04010 Latina (IT)
(72) Inventor: ZAMPIERI, Massimo, I-20052 Monza (IT); AIROLDI, Annalisa, I-20020 Nosate (IT); MARTINI, Alessandro, I-20149 Milano (IT)
(74) Representative: Summers, Victoria Clare
(86) International application number: PCT/EP2003/005261
(87) International publication number: WO 2003/106441

(56) References cited:
- WO-A-01/01973
- GB-A- 2 167 407
- MELLONI P ET AL: "CONFIGURATIONAL STUDIES ON 2-ALPHA-(2-ETHOXYPHENOXY)BENZYLMORPHOLINE FCE 20124" TETRAHEDRON, ELSEVIER SCIENCE PUBLISHERS, AMSTERDAM, NL, vol. 41, no. 7, 1985, pages 1393-1399, XP000989980 ISSN: 0040-4020

## Description

### Field of the invention

The present invention relates to novel crystalline, water-soluble salts of the 2S,3S enantiomer of reboxetine, which are the fumarate and succinate salts thereof, to a process for their preparation, to their utility in therapy and to pharmaceutical compositions containing them.

### Background of the invention

Reboxetine, 2-[α-(2-ethoxy-phenoxy)-benzyl]-morpholine, was first taught by GB 2014981B, which describes its utility for the treatment of depression. Reboxetine is a selective norepinephrine reuptake inhibitor, it is a safe drug and a superior treatment for those disorders in mammals, comprising humans, that need a selective norepinephrine reuptake inhibition. In fact it has few if any physiological effects besides those on norepinephrine processing, and therefore is free of side effects and unwanted activities.

GB 2176407B provides single 2R,3R and 2S,3S enantiomers of reboxetine. The 2S,3S enantiomer of reboxetine, hereafter named as SS-reboxetine, was found to be endowed with a selective norepinephrine reuptake inhibition activity significantly higher that racemate reboxetine.

There are several patent documents describing new uses of reboxetine, for instance US 6,391,876; US 6,046,193; US 6,184,222 US 6,028,070 and WO 02/36125. However the single fumarate and succinate salts of SS-reboxetine have never been described before.

Reboxetine mesylate salt is on the market as racemate and is preferably administered in solid pharmaceutical forms. Similarly, SS-reboxetine mesylate is under development for administration to mammals in solid pharmaceutical forms, which are the most appropriate for administration to patients in need of selective norepinephrine reuptake inhibition. However, compound SS-reboxetine mesylate has shown poor physicochemical characteristics and instability due to its hygroscopicity.

Moisture uptake is a significant concern for pharmaceutical powders. Moisture have been shown to have a significant impact, for example, on the physical, chemical and manufacturing properties of drugs, excipients and formulations. It is also a key factor in taking decisions related to packaging, storage , handling and shelf life and successful development requires a sound understanding of hygroscopic properties.

For instance, conversion from an anhydrous to a hydrate form may be observed when the relative humidity exceeds a critical level and moisture content rapidly increases in the solid. This has not only an impact on the physico-pharmaceutical properties of the drug per se, but also on its biopharmaceutical perspective. Moreover, it is well known, that hydrate forms usually tends to be less soluble with respect to a homologous anhydrous form, with potential detrimental effect also on the dissolution rate properties of the active compound per se and on its absorption profile through the gastrointestinal tract. At the same manner, conversion from a crystalline to an amorphous form may be observed in presence of relative humidity, with potential disadvantages in terms of physical stability (the active drug substance can for instance behave in a deliquescent way) or chemical stability, in fact the amorphous structure being thermodynamically activated is more prone to chemical degradation and to chemical interaction with other chemical species. Thus the performance and the efficacy of both formulation and active ingredient may be significantly changed.

In particular, as far as SS-reboxetine is concerned, it has been ascertained that the anhydrous mesylate salt is per se thermodynamically unstable and tends to transform itself with ageing into a hydrate form. Even more, the anhydrous form tends to lose its crystalline structure while exposed to high relative humidity environment, thus transforming it into a less chemically stable amorphous form.

Accordingly, there is a need in therapy of a water-soluble SS-reboxetine salt endowed with lower hygroscopicity and good and reproducible biopharmaceutical properties for allowing a safer and efficacious oral administration.

The above technical problem has been solved by the inventors of the present invention by providing two novel salts of SS-reboxetine having improved physico-chemical properties. In fact, the novel salts are crystalline, poorly hygroscopic, rapidly-dissolving solids with high water solubility and in addition are substantially more stable than the mesylate salt. They thus possess important advantages in handling, storage and formulations, etc., in addition to possessing all the other advantages, in particular therapeutic advantages, exhibited by the mesylate salt

### Description of the invention

A first object the invention is to provide a novel crystalline, water-soluble salt of 2S,3S enantiomer of 2-[α-(2-ethoxy-phenoxy)-benzyl]-morpholine, which is the fumarate salt and the succinate salt thereof.

2S,3S enantiomer of 2-[α-(2-ethoxy-phenoxy)-benzyl]-morpholine is hereafter named as SS-reboxetine.

Fumarate and succinate salts of SS-reboxetine can be obtained by known analogy methods by means of stoichiometric adding of aqueous solutions of the counterion to the free base dissolved in a suitable solvent. Such solvent is preferably an organic, in particular anhydrous, solvent chosen preferably from methanol, ethanol, dioxane and dimethylformamide. If necessary, the precipitation of the obtained salt may be favoured by adding an anhydrous apolar solvent, for instance diethylether, n-hexane or cyclohexane.

The free SS-reboxetine base can be obtained by the corresponding mesylate salt by known methods The mesylate salt of SS-reboxetine can be obtained as described in GB 2167407B.

According to a preferred feature of the invention, fumarate and succinate salts of SS-reboxetine can be obtained by reacting SS-reboxetine freebase with fumaric acid or succinic acid, respectively, in a suitable lower alkanol preferably ethanol, followed by controlled crystallization process. A lower alkanol is for instance a C1-C4 alkanol, preferably ethanol.

SS-reboxetine freebase in its turn can be obtained by reacting SS-reboxetine mandelate with a suitable basic agent, for instance sodium hydroxide. SS-reboxetine mandelate in its turn can be obtained by reacting reboxetine freebase with (S)-(+)-mandelic acid in a suitable lower alkanol followed by controlled crystallization process. Reboxetine freebase can be obtained by reacting reboxetine mesylate with a suitable basic agent, for instance sodium hydroxide.

Such preferred feature, which is a further object of this invention can be exemplified as follows:

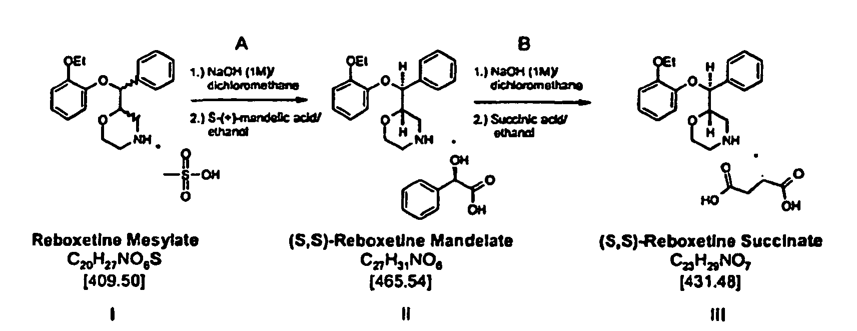

The fumarate and succinate SS-reboxetine salts thus obtained have a crystalline structure.

Object of the invention are also metabolites, metabolic precursors (also known as prodrugs) and hydrate forms of SS-reboxetine fumarate and succinate salts.

A further object of the invention is to provide a pharmaceutical composition comprising a salt of SS-reboxetine, which is the fumarate salt or the succinate salt thereof, as active ingredient and a pharmaceutically acceptable excipient and/or carrier.

A pharmaceutical composition can be formulated according to known method in the art in any of the pharmaceutical forms known in the art for administration to a mammal, including humans. For instance, a pharmaceutical composition containing a compound of the invention, as an active ingredient, and a suitable carrier and/or excipient can be prepared as known from GB 2014981B.

A further object of the invention is to provide a salt of SS-reboxetine, which is the fumarate salt or the succinate salt thereof, for the use as a medicament, in particular as a selective norepinephrine reuptake inhibitor.

A further object of the invention is to provide the use of a salt of SS-reboxetine, which is the fumarate salt or the succinate salt thereof, in the manufacture of a pharmaceutical composition for use in treating a mammal, comprising a human being, suffering from a disease state treatable by selective norepinephrine reuptake inhibition.

Accordingly, the novel SS-reboxetine salts of the invention, either alone or in association with other therapeutic agents, are useful for treating a mammal, comprising humans, suffering from a disease state treatable by selective norepinephrine reuptake inhibition.

The term "disease state treatable" means that the treatment according to the invention provides remission of the disease state or, at least, the conditions and quality of life of the mammal under treatment are improved.

Examples of such disease states are in particular nervous system disorders selected from the group consisting of addictive disorders (including those due to alcohol, nicotine, and other psychoactive substances) and withdrawal syndrome, adjustment disorders (including depressed mood, anxiety, mixed anxiety and depressed mood, disturbance of conduct, and mixed disturbance of conduct and mood), age-associated learning and mental disorders (including Alzheimer's disease), anorexia nervosa, apathy, attention-deficit (or other cognitive) disorders due to general medical conditions, attention-deficit hyperactivity disorder (ADHD), bipolar disorder, bulimia nervosa, chronic fatigue syndrome, chronic or acute stress, chronic pain, neuropathic pain, neuralgias including postherpatic neuralgias, conduct disorder, cyclothymic disorder, depression (including refractory depression, adolescent depression and minor depression), dysthymic disorder, fibromyalgia and other somatoform disorders (including somatization disorder, conversion disorder, pain disorder, hypochondriasis, body dysmorphic disorder, undifferentiated somatoform disorder, and somatoform NOS), generalized anxiety disorder (GAD), incontinence (*i.e*., stress incontinence, genuine stress incontinence, and mixed incontinence), inhalation disorders, intoxication disorders (alcohol addiction), mania, migraine headaches, obesity (*i.e*., reducing the weight of obese or overweight patients), obsessive compulsive disorders and related spectrum disorders, oppositional defiant disorder, panic disorder, peripheral neuropathy, diabetic neuropathy, post-traumatic stress disorder, premenstrual dysphoric disorder (*i.e*., premenstrual syndrome and late luteal phase dysphoric disorder), psychotic disorders (including schizophrenia, schizoaffective and schizophreniform disorders), seasonal affective disorder, sleep disorders (such as narcolepsy and enuresis), social phobia (including social anxiety disorder), specific developmental disorders, selective serotonin reuptake inhibition (SSRJ) "poop out" syndrome (*i.e*., wherein a patient who fails to maintain a satisfactory response to SSRI therapy after an initial period of satisfactory response), and TIC disorders (*e.g*., Tourette's Disease). As stated above, the novel SS-reboxetine salts of the invention can be used also in association with other therapeutic agents, for instance with pindolol for fast onset of antidepressant activity, with detrol for incontinence, and with a neuroleptic agent , e.g. a typical or atypical antipsychotic agent, to treat schizophrenia.

The effective dose of SS-reboxetine fumarate or SS-reboxetine succinate salt may vary according to the disease, severity of the disorder and the conditions of the patient to be treated. Therefore the optimal dose for each patient, as always, must be set by the physician. Anyway, the effective dosage range may be from about 0.5 mg/day to about 20 mg/day, preferably from about 1 to about 15 mg/day (calculated as free base), either as a single or multiple divided daily dosages.

SS-reboxetine fumarate and SS-reboxetine succinate are readily orally absorbed, therefore they are preferably orally administered. Anyway, they may be administered by any administration route, for instance by parenteral, topical, rectal and nasal route.

The following examples illustrate the invention.

### Example 1

### Preparation of Reboxetine S,S enantiomer Succinate salt

Succinate salt of S,S-reboxetine has been synthesized by adding a stoichiometric amount of succinic acid to the ethanolic solution of the free base. 16 ml of a methanolic solution containing 2.5 g of succinic acid has been added to 4.1 g of free base (yellow-orange oil) dissolved in 75 ml of absolute ethanol.

The solution was then heated under stirring at 40°C for about 20 minutes. The solution has become colorless and a white, fine precipitate was observed. The yield of crystallization was then forced by cooling the slurry at -30°C to facilitate the salt formation.

The solid was then separated by vacuum filtration and dried about 8 hours under vacuum at 40°C. At the above conditions here described, the succinate salt of S,S-reboxetine was obtained.

### Example 2

### Preparation of Reboxetine S,S enantiomer Fumarate salt

Fumarate salt of S,S-reboxetine has been synthesized by means of the same stoichiometric crystallization technique described above. 1.6 g of fumaric acid suspended in 10 ml of absolute ethanol has been added to 4.1 g of free base dissolved in 75 ml of absolute ethanol.

The solution was then heated under stirring at 40°C for a few minutes. At once formation of white-rose, spherical agglomerates was observed. The yield of crystallization was then forced by cooling the slurry at -30°C to facilitate the salt formation. The solid was then separated by vacuum filtration and dried about 8 hours under vacuum at 40°C. By means of the below procedure, the fumarate salt of S,S-reboxetine was obtained.

### Example 3

### Preparation of Reboxetine S,S enantiomer Succinate salt

Step A: Freebase reboxetine mesylate with aqueous sodium hydroxide into a dichloromethane phase. Evaporate dichloromethane from the reboxetine freebase and add ethanol. Dissolve 1.1 equivalents of (S)-(+)-mandelic acid in ethanol. Mix the freebase and acid solutions to form a precipitate of (S,S)-reboxetine mandelate following a controlled crystallization process. Isolate the solids by filtration and drying. Upgrade chiral purity of the (S,S)-reboxetine by reflux and recrystallization in ethanol. Isolate solids again by filtration and drying.

Step B: Freebase (S,S)-reboxetine mandelate with aqueous sodium hydroxide into a dichloromethane phase. Evaporate dichloromethane from the reboxetine freebase and add ethanol. Dissolve 1.0 equivalents of succinic acid in ethanol. Mix the freebase and acid solutions to form a precipitate of (S,S)-reboxetine succinate following a controlled crystallization process. Isolate the solids by filtration and drying.

### Analytical Results

### X-ray powder diffraction (XRD)

The SS-reboxetine fumarate and SS-reboxetine succinate salts were characterized by X-ray powder diffraction (XRP), as follows:

Powder X-ray diffraction was performed using a Siemens D-500 apparatus, irradiating powder samples with a CuKα graphite-monochromatic (40 kV 40 mA) source between 5° and 35° (2θ) at room temperature. The scan was made of 0.05° steps and the count time was 7 seconds per step.

The main X-ray diffraction peaks of succinate and fumarate salts are here below summarized in the following Table I (succinate salt) and Table II (fumarate salt).

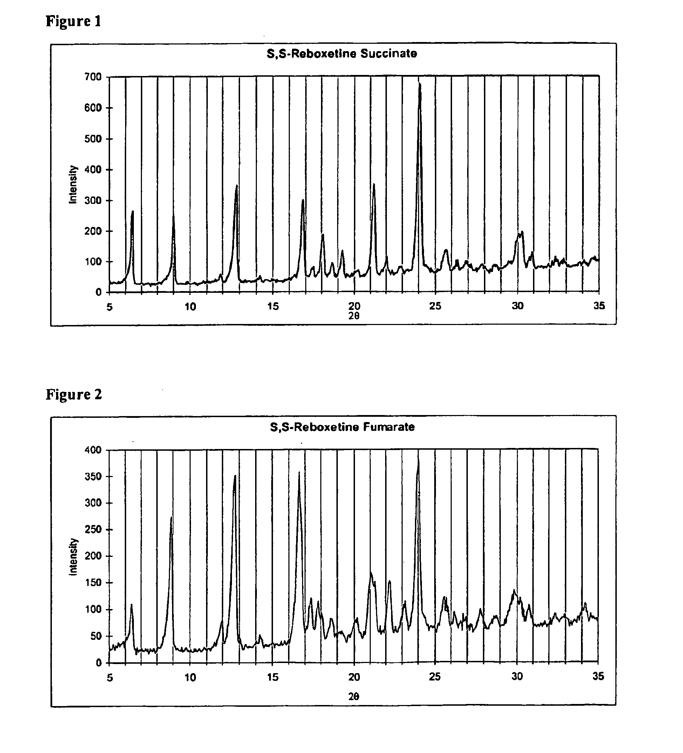

The relevant spectra are reported in Figures 1 and 2.

**Table I**

| **Angle (°2θ)** | **Relative Intensity** |
|---|---|
| 6.45 | 39.3 |
| 9.00 | 37.9 |
| 12.85 | 51.5 |
| 16.85 | 44.7 |
| 18.10 | 27.7 |
| 19.30 | 19.8 |
| 21.20 | 51.9 |
| 22.05 | 16.9 |
| 24.05 | 100.0 |
| 25.70 | 20.1 |
| 30.10 | 27.7 |
| 30.30 | 29.0 |
| 30.90 | 18.9 |

**Table II**

| **Angle (°2θ)** | **Relative Intensity** |
|---|---|
| 6.40 | 28.9 |
| 8.90 | 71.9 |
| 12.75 | 92.1 |
| 16.65 | 94.0 |
| 17.40 | 31.5 |
| 17.85 | 30.2 |
| 21.30 | 39.9 |
| 22.25 | 40.1 |
| 23.20 | 29.9 |
| 24.05 | 100 |
| 25.60 | 32.0 |
| 25.70 | 31.7 |
| 29.85 | 35.4 |

### Differential Scanning Calorimetry (DSC)

DSC analyses were carried out with a Perkin-Elmer DSC-7 apparatus. Aluminium DSC pans were loaded with about of 2 mg of sample. The temperature range of analysis was between 30° and 210°C. The samples were analyzed under nitrogen flow (to eliminate oxidative and pyrolitic effects) at a heating rate of 10°C/min.

For succinate salt the observed melting endotherm was at approximately 148°C [heat fusion (ΔH_{f}) approximately 120 J/g]. The melting endotherm of fumarate salt was at approximately 171°C [heat fusion (ΔH_{f}) approximately 100 J/g].

### Stability data

Solid state of succinate and fumarate salts has been controlled after an accelerated stability plan. The samples were conserved for 2 weeks at 65°C in glasses HPLC vials and then controlled by means of DSC.

No changes in solid state were observed for both the samples.

### Solubility

The determination of water solubility of succinate and fumarate salts of S,S-reboxetine has been performed by means of the following procedure: an excess solid (in order to have saturated solutions) has been added into a vial to 1.5 ml of water. The vials were stirred mechanically shaken at 37°C. At appropriate time (i.e. 1 hour) samples were withdrawn and solubility assayed by means of a specific HPLC assay.

The results are here below summarized in Table III.

**Table III**

| Sample | Aqueous solubility (mg/ml) | | |
|---|---|---|---|
| | 1 b stirring | 2 hrs stirring | 24 hrs stirring |
| Succinate salt | 30 | 30 | 35 |
| Fumarate salt | 9 | 9 | 9 |

### Dynamic Moisture Sorption Gravimetry (DMSG)

The water uptake of succinate and fumarate salts of S,S-reboxetine was investigated by submitting a sample of such salts to a hygroscopicity test by means of a DVS 1000 (SMS) according to the principle of Dynamic Moisture Sorption Gravimetry (DMSG). The apparatus is a "controlled atmosphere microbalance" where the weighed sample is exposed to programmed variations of the relative humidity (RH) at a constant and controlled temperature. The measured parameters (weight, time and RH), reported in Excel worksheets, allowed obtaining hygroscopicity curves over the tested RH range. Multiple sorption/desorption cycles between 0% and 90% RH were performed at 25°C. Progressive variations of RH were of 10%; they were operated by the software at the equilibration of the sample weight. This condition was defined at a constant rate of percent weight variation 0.005%/min (average of 5 minutes survey). The experimental results were reported in the DVS Isotherm Reports and Isotherm Plots.

The water uptake of succinate and fumarate salts of S,S-reboxetine is here below summarized in the following Table IV.

**Table IV**

| **Relative Humidity** (%) | **Succinate salt Water uptake %** | **Fumarate salt Water uptake %** |
|---|---|---|
| 20 | 0.03 | 0.06 |
| 35 | 0.06 | 0.11 |
| 50 | 0.10 | 0.16 |
| 65 | 0.14 | 0.21 |
| 80 | 0.21 | 0.31 |
| 90 | 0.29 | 0.47 |

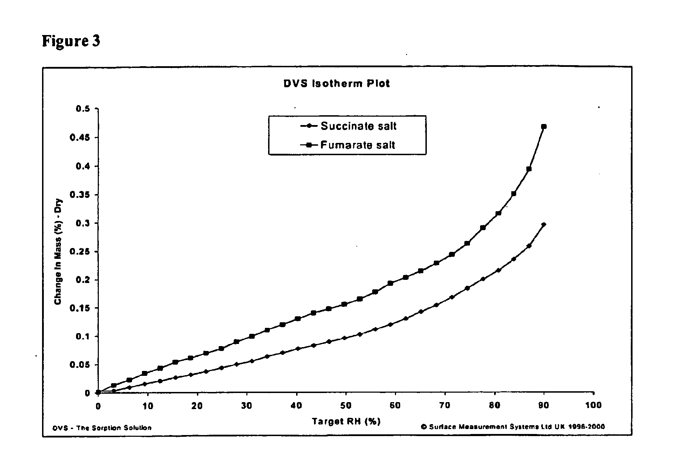

The sorption profiles of the two salts are shown in Figure 3. The water uptake observed is anyway reversible, thus non altering the chemical, physico-chemical and solid state characteristics of both fumarate and succinate salts.

For comparison purposes also solid state characterization of S,S-reboxetine mesylate was characterized by means of the techniques described above.

### Differential Scanning Calorimetry (DSC)

The melting point, determined by means of DSC analyses measuring the endothermic feature related to sample fusion, was about 106°C.

### Thermogravimetric Analysis (TGA)

The volatile content measured by means of Thermogravimetric Analysis (TGA) was relevant: in fact, a weight loss of about 2% was detected upon heating showing a related thermal feature. Whereas for SS-reboxetine fumarate and succinate salts a negligible weight loss was measured.

### Dynamic Moisture Sorption Gravimetry (DMSG)

During DVS analyses, similar to those previously described, this compound showed a relevant tendency to moisture uptake. The amount of water taken up by the sample after the DVS sorption step was only partially eliminated by decreasing relative humidity and solid state modification was observed by means of DSC analyzing the tested sample.

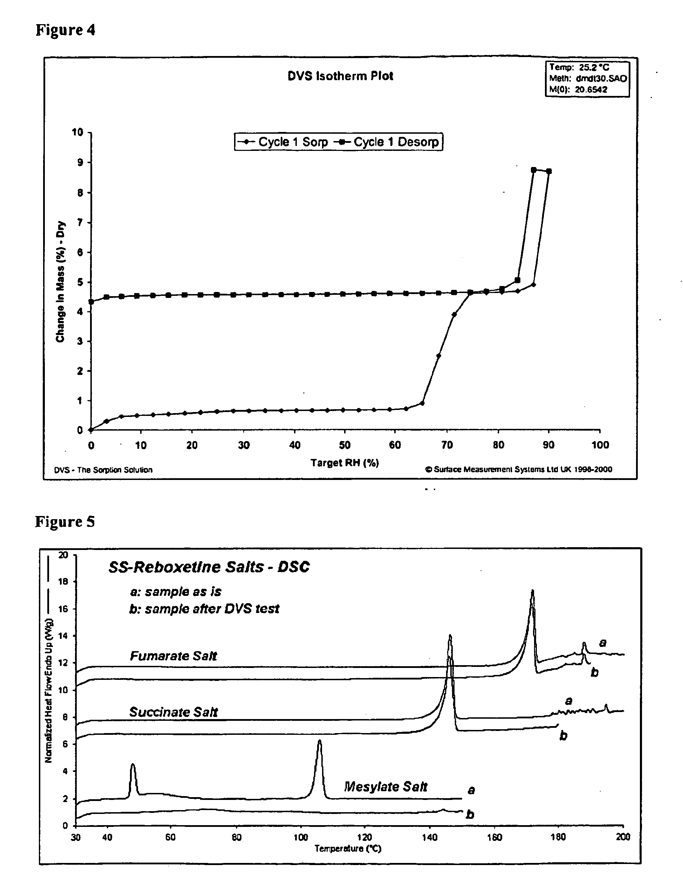

The obtained results are summarized in the Table V, reporting the water uptake expressed as percent change in mass, and Figure 4.

**Table V**

| **Relative Humidity (%)** | **Sorption Cycle Water content %** | **Desorption Cycle Water content %** |
|---|---|---|
| 0 | 0.0 | 4.3 |
| 20 | 0.5 | 4.5 |
| 35 | 0.6 | 4.6 |
| 50 | 0.7 | 4.6 |
| 65 | 0.9 | 4.6 |
| 80 | 4.5 | 4.7 |
| 90 | 8.7 | 8.7 |

The above comparative testing results, obtained through the main analytical techniques to characterise succinate, fumarate and mesylate salts of S,S-reboxetine, are here below summarize.

### Dynamic Moisture Sorption Gravimetry

Hygroscopicity tests operated by means of a DVS 1000 (SMS) according to the principle of Dynamic Moisture Sorption Gravimetry (DMSG) before reported, show that mesylate salt tends to adsorb a high amount of water (up to 9 % at 90% RH) while the uptakes of the new succinate and fumarate salts below 0.5 %. Furthermore mesylate salt retains about half of the detected uptake (about 4 %) also after re-equilibration and show modification of solid structure (loss of crystallinity observed by DSC).

The comparison between the behaviour of the different salts in the presence of moisture can be summarized as here below in Table VI (the relevant raw data are above shown in Tables 3 and 4, and Figures 3 and 4)

**Table VI**

| **Salt** | **Succinate salt** | **Fumarate salt** | **Mesylate salt** |
|---|---|---|---|
| Effects of moisture uptake → | Reversible water uptake dependent on Relative Environmental Humidity. No changes in crystalline form (**Figure 3**) | Reversible water uptake dependent on Relative Environmental Humidity. No changes in crystalline form (**Figure 3**) | Irreversible change in crystalline for due to water uptake. Retention of water even when the drug is re-exposed at lower humidities (**Figure 4**) |

### Differential Scanning Calorimetry (DSC)

DSC analyses were executed as reported above also on the samples recovered after DVS tests, executed according to the DMSG principle. As shown below in Figure 5, DSC profiles of SS-reboxetine succinate and fumarate salts were unchanged after equilibration at high humidity according to the DVS test method (maximum relative humidity of 90% at 25°C and equilibration up to 0.005%/min or no more than 360 minutes).

On the other hand, mesylate salt was affected by a complete destructuration indicated by the disappearence of the main thermal feature (dehydration at about 50°C and melting at about 105°C).

From the above comparative data the person skilled in the art will appreciate that the new salts of the invention are an improved and valuable new tool in therapy.

## Claims

1. A salt of 2S, 3S enantiomer of 2-[α-(2-ethoxy-phenoxy)-benzyl]-morpholine, which is the fumarate salt or the succinate salt thereof.

2. A salt as claimed in claim 1, which is the fumarate salt.

3. A salt as claimed in claim 1, which is the succinate salt.

4. A salt according to Claim 3 wherein the salt is crystalline.

5. A salt according to Claim 4 which is **characterised by** a powder X-ray diffraction pattern (PXRD) which shows main peaks at 12.85, 16.85, 21.20, and 24.05 degrees 2θ.

6. A salt according to Claim 5 wherein the salt has further powder X-ray diffraction pattern (PXRD) peaks at 6.45, 9.00, 18.10, 30.10 and 30.30 degrees 2θ.

7. A salt according to Claim 6 wherein the salt has further powder X-ray diffraction pattern (PXRD) peaks at 19.30, 22.05, 25.70 and 30.90 degrees 2θ.

8. A salt according to Claim 4 wherein the differential scanning calorimetry (DSC) trace shows a sharp endotherm at 148°C.

9. A salt according to Claim 2 wherein the salt is crystalline.

10. A salt according to Claim 9 which is **characterised by** a powder X-ray diffraction pattern (PXRD) which shows main peaks at 8.90, 12.75, 16.65 and 24.05 degrees 2θ.

11. A salt according to Claim 10 wherein the salt has further powder X-ray diffraction pattern (PXRD) peaks at 6.40, 17.40, 17.85, 21.30, 22.25, 23.20, 25.60, 25.70 and 29.85 degrees 2θ.

12. A salt according to Claim 9 wherein the differential scanning calorimetry (DSC) trace shows a sharp endotherm at 171°C.

13. A pharmaceutical composition comprising a salt, as claimed in claim 1, as active ingredient and a pharmaceutically acceptable excipient and/or carrier.

14. A salt as claimed in claim 1, for use as a medicament.

15. A salt as claimed in claim 1, for use as a selective norepinephrine reuptake inhibitor.

16. Use of a salt as claimed in claim 1, in the manufacture of a pharmaceutical composition for use in treating a mammal, including humans, suffering from a disease state treatable by selective norepinephrine reuptake inhibition.

17. Use as claimed in claim 16, wherein the disease state is selected from the group consisting of attention-deficit (or other cognitive) disorders due to general medical conditions, chronic fatigue syndrome, chronic pain, neuropathic pain, neuralgias, fibromyalgia and other somatoform disorders, incontinence, migraine headaches, obesity, and peripheral neuropathy.

18. Use as claimed in claim 17, wherein the disease state is fibromyalgia and other somatoform disorders.

19. A salt, as claimed in Claim 1, for the treatment of a mammal, including humans, suffering from a disease state treatable by selective norepinephrine reuptake inhibition.

20. A salt, as claimed in Claim 19, for the treatment of a mammal, including humans, suffering from a disease state, wherein the disease state is selected from the group consisting of attention-deficit (or other cognitive) disorders due to general medical conditions, chronic fatigue syndrome, chronic pain, neuropathic pain, neuralgias, fibromyalgia and other somatoform disorders, incontinence, migraine headaches, obesity, and peripheral neuropathy.

21. A salt, as claimed in Claim 20, for the treatment of a mammal, including humans, suffering from a disease state, wherein the disease state is fibromyalgia and other somatoform disorders.

22. A process for the preparation of a salt of 2S, 3S enantiomer of 2-[α-(2-ethoxy-phenoxy)-benzyl]-morpholine, which is the fumarate salt or the succinate salt thereof, which comprises: reacting 2-[α-(2-ethoxy-phenoxy)-benzyl]-morpholine with (S) (+) mandelic acid so obtaining 2S, 3S 2-[α-(2-ethoxy-phenoxy)-benzyl]-morpholine mandelate; reacting 2S, 3S 2-[α-(2-ethoxy-phenoxy)-benzyl]-morpholine mandelate; with a suitable basic agent so obtaining the corresponding free base; and reacting 2S, 3S 2-[α-(2-ethoxy-phenoxy)-benzyl]-morpholine, with fumaric acid or succinic acid, respectively, followed by a controlled crystallization process.

## Patentansprüche

1. Salz des 2S,3S-Enantiomers von 2-[α-(2-Ethoxyphenoxy)benzyl]morpholin, welches das Fumaratsalz oder das Succinatsalz desselben ist.

2. Salz gemäß Anspruch 1, welches das Fumaratsalz ist.

3. Salz gemäß Anspruch 1, welches das Succinatsalz ist.

4. Salz gemäß Anspruch 3, wobei das Salz kristallin ist.

5. Salz gemäß Anspruch 4, das durch ein Pulverröntgenbeugungsspektrum ("powder X-ray diffraction pattern" (PXRD)) **gekennzeichnet** ist, welches Hauptpeaks bei 12,85, 16,85, 21,20 und 24,05 Grad 2θ zeigt.

6. Salz gemäß Anspruch 5, wobei das Salz außerdem Pulverröntgenbeugungsspektrum (PXRD)-Peaks bei 6,45, 9,00, 18,10, 30,10 und 30,30 Grad 2θ hat.

7. Salz gemäß Anspruch 6, wobei das Salz außerdem Röntgenbeugungsmuster (PXRD)-Peaks bei 19,30, 22,05, 25,70 und 30,90 Grad 2θ hat.

8. Salz gemäß Anspruch 4, wobei die Differential-Scanning-Kalorimetrie (DSC)-Kurve eine scharfe Endotherme bei 148°C zeigt.

9. Salz gemäß Anspruch 2, wobei das Salz kristallin ist.

10. Salz gemäß Anspruch 9, welches durch ein Pulverröntgenbeugungsspektrum (PXRD) **gekennzeichnet** ist, das Hauptpeaks bei 8,90, 12,75, 16,65 und 24,05 Grad 2θ zeigt.

11. Salz gemäß Anspruch 10, wobei das Salz außerdem Pulverröntgenbeugungsspektrum (PXRD)-Peaks bei 6,40, 17,40, 17,85, 21,30, 22,25, 23,20, 25,60, 25,70 und 29,85 Grad 2θ hat.

12. Salz gemäß Anspruch 9, wobei die Differential-Scanning-Kalorimetrie (DSC)-Kurve eine scharfe Endotherme bei 171°C zeigt.

13. Pharmazeutische Zusammensetzung, umfassend ein Salz gemäß Anspruch 1 als aktives Ingredienz und ein pharmazeutisch verträgliches Exzipiens und/oder einen pharmazeutisch verträglichen Träger.

14. Salz gemäß Anspruch 1 zur Verwendung als Medikament.

15. Salz gemäß Anspruch 1 zur Verwendung als selektiver Norepinephrin-Wiederaufnahmeinhibitor.

16. Verwendung eines Salzes gemäß Anspruch 1 bei der Herstellung einer pharmazeutischen Zusammensetzung zur Verwendung bei der Behandlung eines Säugers, einschließlich Menschen, der an einem Erkrankungszustand leidet, der durch selektive Norepinephrin-Wiederaufnahmeinhibition behandelbar ist.

17. Verwendung gemäß Anspruch 16, wobei der Erkrankungszustand aus der Gruppe, bestehend aus Aufmerksamskeitsstörungen (oder anderen kognitiven Störungen) in Folge allgemeiner medizinischer Zustände, chronischem Müdigkeitssyndrom, chronischem Schmerz, neuropathischem Schmerz, Neuralgien, Fibromyalgie und anderen somatoformen Störungen, Inkontinenz, Migräne, Kopfschmerzen, Adipositas und peripherer Neuropathie ausgewählt ist.

18. Verwendung gemäß Anspruch 17, wobei der Erkrankungszustand Fibromyalgie und andere somatoforme Störungen ist.

19. Salz gemäß Anspruch 1 für die Behandlung eines Säugers, einschließlich Menschen, der an einem Erkrankungszustand leidet, der durch selektive Norepinephrin-Wiederaufnahmeinhibition behandelbar ist.

20. Salz gemäß Anspruch 19 für die Behandlung eines Säugers, einschließlich Menschen, der an einem Erkrankungszustand leidet, wobei der Erkrankungszustand ausgewählt ist aus der Gruppe, bestehend aus Aufmerksamkeitsstörungen (oder anderen kognitiven Störungen) in Folge allgemeiner medizinischer Zustände, chronischem Müdigkeitssyndrom, chronischem Schmerz, neuropathischem Schmerz, Neuralgien, Fibromyalgie und anderen somatoformen Störungen, Inkontinenz, Migräne, Kopfschmerzen, Adipositas und peripherer Neuropathie ausgewählt ist.

21. Salz gemäß Anspruch 20 für die Behandlung eines Säugers, einschließlich Menschen, der an einem Erkrankungszustand leidet, wobei der Erkrankungszustand Fibromyalgie und andere somatoforme Störungen ist.

22. Verfahren für die Herstellung eines Salzes des 2S,3S-Enantiomers von 2-[α-(2-Ethoxyphenoxy)benzyl]morpholin, welches das Fumaratsalz oder das Succinatsalz desselben ist, welches umfasst: Umsetzen von 2-[α-(2-Ethoxyphenoxy)benzyl]morpholin mit (S)-(+)-Mandelsäure unter Erhalt von 2S,3S-2-[α-(2-Ethoxyphenoxy)benzyl]morpholin-Mandelat; Umsetzen von 2S,3S-2-[α-(2-Ethoxyphenoxy)benzyl]morpholin-Mandelat mit einem geeigneten basischen Mittel unter Erhalt der entsprechenden freien Base und Umsetzen von 2S,3S-2-[α-(2-Ethoxyphenoxy)benzyl]morpholin mit Fumarsäure oder Bernsteinsäure, gefolgt von einem Verfahren der kontrollierten Kristallisation.

## Revendications

1. Sel d'énantiomère 2S, 3S de 2-[α-(2-éthoxyphénoxy)benzyl]morpholine, qui est le sel consistant en fumarate ou sel consistant en succinate de cet énantiomère.

2. Sel suivant la revendication 1, qui est le fumarate.

3. Sel suivant la revendication 1, qui est le succinate.

4. Sel suivant la revendication 3, qui est cristallin.

5. Sel suivant la revendication 4, qui est **caractérisé par** un diagramme de diffraction des rayons X sur poudre (PXRD) qui présente des pics principaux à 12,85, 16,85, 21,20 et 24,05 degrés 2θ.

6. Sel suivant la revendication 5, qui a des pics supplémentaires dans le diagramme de diffraction des rayons X sur poudre (PXRD) à 6,45, 9,00, 18,10, 30,10 et 30,30 degrés 2θ.

7. Sel suivant la revendication 6, qui a des pics supplémentaire dans le diagramme de diffraction des rayons X sur poudre (PXRD) à 19,30, 22,05, 25,70 et 30,90 degrés 2θ.

8. Sel suivant la revendication 4, dans lequel le graphique de calorimétrie à balayage différentiel (DSC) présente un comportement endothermique net à 148°C.

9. Sel suivant la revendication 2, qui est cristallin.

10. Sel suivant la revendication 9, qui est **caractérisé par** un diagramme de diffraction des rayons X sur poudre (PXRD) qui présente des pics principaux à 8,90, 12,75, 16,65 et 24,05 degrés 2θ.

11. Sel suivant la revendication 10, qui présente des pics supplémentaires dans le diagramme de diffraction des rayons X sur poudre (PXRD) à 6,40, 17,40, 17,85, 21,30, 22,25, 23,20, 25,60, 25,70 et 29,85 degrés 2θ.

12. Sel suivant la revendication 9, dans lequel le graphique de calorimétrie à balayage différentiel (DSC) présente un comportement endothermique net à 171°C.

13. Composition pharmaceutique comprenant un sel suivant la revendication 1, comme ingrédient actif, et un excipient et/ou support pharmaceutiquement acceptable.

14. Sel suivant la revendication 1, destiné à être utilisé comme médicament.

15. Sel suivant la revendication 1, destiné à être utilisé comme inhibiteur sélectif de réabsorption de norépinéphrine.

16. Utilisation d'un sel suivant la revendication 1, dans la production d'une composition pharmaceutique destinée à être utilisée dans le traitement d'un mammifère, y compris d'êtres humains, souffrant d'un état pathologique pouvant être traité par inhibition sélective de la réabsorption de norépinéphrine.

17. Utilisation suivant la revendication 16, dans laquelle l'état pathologique est choisi dans le groupe consistant en des troubles de déficit d'attention (ou d'autres troubles cognitifs) dus à un état médical général, le syndrome de fatigue chronique, la douleur chronique, la douleur neuropathique, les névralgies, la fibromyalgie et d'autres troubles somatoformes, l'incontinence, les céphalées dues à la migraine, l'obésité et la neuropathie périphérique.

18. Utilisation suivant la revendication 17, dans laquelle l'état pathologique est la fibromyalgie ou un autre trouble somatoforme.

19. Sel suivant la revendication 1 pour le traitement d'un mammifère, y compris d'êtres humains, souffrant d'un état pathologique pouvant être traité par inhibition sélective de la réabsorption de norépinéphrine.

20. Sel suivant la revendication 19, pour le traitement d'un mammifère, y compris d'êtres humains souffrant d'un état pathologique, dans lequel l'état pathologique est choisi dans le groupe consistant en des troubles de déficit d'attention (ou d'autres troubles cognitifs) dus à un état médical général, le syndrome de fatigue chronique, la douleur chronique, la douleur neuropathique, les névralgies, la fibromyalgie, et d'autres troubles somatoformes, l'incontinence, les céphalées dues à la migraine, l'obésité et la neuropathie périphérique.

21. Sel suivant la revendication 20, pour le traitement d'un mammifère, y compris d'êtres humains souffrant d'un état pathologique, dans lequel l'état pathologique est la fibromyalgie ou un autre trouble somatoforme.

22. Procédé pour la préparation d'un sel d'énantiomère 2S,3S de 2-[α-(2-éthoxyphénoxy)benzyl]morpholine, qui est le sel consistant en fumarate ou le sel consistant en succinate de cet énantiomère, qui comprend : la réaction de 2-[α-(2-éthoxyphénoxy)benzyl]morpholine avec de l'acide (S)-(+)-mandélique, ce qui permet d'obtenir le mandélate de 2S,3S-2-[α-(2-éthoxyphénoxy)benzyl]morpholine ; la réaction du mandélate de 2S,3S-2-[α-(2-éthoxyphénoxy)benzyl]-morpholine avec un agent basique convenable, ce qui permet d'obtenir la base libre correspondante ; et la réaction de la 2S,3S-2-[α-(2-éthoxyphénoxy)benzyl]morpholine avec de l'acide fumarique ou de l'acide succinique, respectivement, avec ensuite un procédé de cristallisation contrôlée.
